# EUROPEAN PATENT SPECIFICATION

(11) **EP 4 282 450 B1**
(45) Date of publication and mention of the grant of the patent: **29.10.2025**
(21) Application number: 23171280.3
(22) Date of filing: 03.05.2023
(51) Int. Cl.: A61M 5/142, A61M 5/145

(54) **DRIVING MECHANISM FOR DRIVING A PLUNGER OF AN AUTO-INJECTOR TO SLIDE RELATIVE TO A RESERVOIR OF THE AUTO-INJECTOR AND AUTO-INJECTOR THEREWITH**
ANTRIEBSMECHANISMUS ZUM ANTREIBEN EINES KOLBENS EINES AUTOINJEKTORS ZUM GLEITEN RELATIV ZU EINEM RESERVOIR DES AUTOINJEKTORS UND AUTOINJEKTOR DAMIT
MÉCANISME D'ENTRAÎNEMENT POUR ENTRAÎNER UN PISTON D'UN AUTO-INJECTEUR À GLISSER PAR RAPPORT À UN RÉSERVOIR DE L'AUTO-INJECTEUR ET AUTO-INJECTEUR AVEC CELUI-CI

(30) Priority: 22.05.2022 US 202263344631 P; 23.03.2023 US 202318125129
(43) Date of publication of application: 29.11.2023
(73) Proprietor: Altek Biotechnology Corporation, 300 Hsinchu City (TW)
(72) Inventor: Huang, Yu-Cheng, 300 Hsinchu City (TW)
(74) Representative: Straus, Alexander

(56) References cited:
- US-A1- 2004 064 088
- US-A1- 2016 038 691
- US-A1- 2022 143 304

## Description

### Field of the Invention

The present invention relates to a driving mechanism and an auto-injector therewith according to the pre-characterizing clauses of claims 1 and 15.

### Background of the Invention

An auto-injector, e.g., an on-body injector, is a medical device designed to deliver a dose of a drug. However, the conventional auto-injectors available in the markets are unable to meet requirements of small volume, high driving power, long driving distance, long injecting period and accurate drug dose delivery rate. Therefore, an improvement of the auto-injector is urgently needed.

For example, in US 2004/0640088 A1, it discloses a device for delivering fluid. The device includes an exit port assembly, a reservoir having a side wall extending towards an outlet connected to the exit port assembly, a threaded lead screw received at least partly in the reservoir and longitudinally extending towards the outlet, and a plunger secured to the lead screw and having an outer periphery linearly slidable along the side wall of the reservoir. A tube is coaxially received on the lead screw and includes a longitudinal slot. A pin extends through the lead screw and the slot of the tube. A gear is secured to the tube for rotation therewith.

Besides, in US 2022/0143304 A1, it discloses an infusion device. The infusion device includes an infusion unit having a drug storage unit, a metal piece, a piston, a rigid screw, a rotating shaft, a driving unit, a driving wheel provided with wheel teeth, a power unit and a rebound unit. The driving unit includes at least two driving portions. The driving unit can rotate around the rotating shaft in the driving direction and the returning direction. The power unit and the rebound unit cooperate with each other to apply force to the driving unit to rotate the driving unit. The infusion device also includes a position detector. The metal piece and the position detector interact to generate signals.

Furthermore, in US 2016/0038691 A1, it discloses a delivery device having two second housing portions that selectively engage and disengage. A reservoir on one of the two housing portions operatively engages a drive device and/or a needle inserting device on the other one of the two housing portions. Upon proper engagement of the two housing portions, the reservoir operatively couples to the drive device and/or the needle inserting device. A first magnet and a second magnet are respectively positioned on the two housing portions to magnetically interact with each other, upon operative engagement of the housing portions. A third magnet is opposed to the first magnet to help align the two housing portions for connection. A magnet-responsive device is used to detect alignment and/or connection of the housing portions.

### Summary of the Invention

This in mind, the present invention aims at providing a driving mechanism for driving a plunger of an auto-injector to slide relative to a reservoir of the auto-injector and an auto-injector therewith.

This is achieved by a driving mechanism and an auto-injector therewith according to claims 1 and 15. The dependent claims pertain to corresponding further developments and improvements.

As will be seen more clearly from the detailed description following below, the claimed driving mechanism is for driving a plunger of an auto-injector to slide relative to a reservoir of the auto-injector. The driving mechanism includes a first transmission component, a driving component, a second transmission component, a driving resilient component, a stopping resilient component, a third transmission component, a sliding component and a supporting component. The driving component is coupled to the first transmission component and for driving the first transmission component to rotate. The second transmission component is rotatably disposed apart from the first transmission component. The driving resilient component is arranged between the first transmission component and the second transmission component. The driving resilient component is configured to be forced by the first transmission component to resiliently deform to push the second transmission component to rotate along a first rotating direction, or the driving resilient component is configured to be released to resiliently recover. The stopping resilient component is disposed adjacent to an outer periphery of the second transmission component. The stopping resilient component is forced by the second transmission component to resiliently deform when the driving resilient component is forced by the first transmission component to resiliently deform to push the second transmission component to rotate along the first rotating direction. The stopping resilient component engages with the second transmission component for stopping the second transmission component from rotating along a second rotating direction opposite to the first rotating direction when the driving resilient component is released to resiliently recover. The third transmission component is fixedly connected to the second transmission component. The third transmission component is driven by the second transmission component to rotate together with the second transmission component when the second transmission component rotates. The sliding component is at least partially slidably disposed inside the third transmission component and movably engaged with the third transmission component. The sliding component is connected to the plunger. The sliding component is driven by the third transmission component to slide relative to the second transmission component along a first sliding direction when the third transmission component is driven by the second transmission component to rotate along the first sliding direction together with the second transmission component. The supporting component includes a guiding portion. The sliding component passes through the guiding portion, and the guiding portion is configured to guide the sliding component to slide along the first sliding direction without rotation.

According to an embodiment of the present invention, the driving mechanism further includes a sensor configured to sense a rotating movement of the second transmission component.

According to an embodiment of the present invention, the sensor includes an abutting component. The abutting component is abutted by the stopping resilient component when the stopping resilient component is resiliently deformed by the second transmission component.

According to an embodiment of the present invention, the driving resilient component and the stopping resilient component are integrally connected to each other to form an integral resilient structure.

According to an embodiment of the present invention, the supporting component further includes a mounting portion for mounting the integral resilient structure.

According to an embodiment of the present invention, a channel is formed on the mounting portion. The integral resilient structure passes through the channel, and the driving resilient component and the stopping resilient component are partially exposed out of the mounting portion.

According to an embodiment of the present invention, the driving resilient component is resiliently deformed by the first transmission component along a first deforming direction identical to the first rotating direction, and the stopping resilient component is resiliently deformed by the second transmission component along a second deforming direction opposite to the first deforming direction.

According to an embodiment of the present invention, the first transmission component, the second transmission component and the third transmission component are accommodated inside the supporting component.

According to an embodiment of the present invention, a first rotating axis of the first transmission component is parallel to a second rotating axis of the second transmission component, and the first sliding direction is parallel to an extending direction of the second rotating axis of the second transmission component.

According to an embodiment of the present invention, the first transmission component is a cam component. The second transmission component is a ratchet component. The third transmission component is a screw sleeve. The sliding component is a screw rod, and the driving component is an electric motor.

According to an embodiment of the present invention, the driving mechanism further includes a reducer coupled between the driving component and the first transmission component.

According to an embodiment of the present invention, the reducer is a gearbox.

According to an embodiment of the present invention, the guiding portion includes a sliding through hole structure. The sliding component slidably passes through the sliding through hole structure. A cross section of the sliding component matches with a cross section of the sliding through hole structure. The sliding component includes at least one first arc part and at least one first flat part connected to the at least one first arc part. The sliding through hole structure includes at least one second arc part and at least one second flat part connected to the at least one second arc part, and the at least one second arc part and the at least one second flat part are arranged respectively corresponding to the at least one first arc part and the at least one first flat part.

According to an embodiment of the present invention, an internal thread structure is formed on an inner periphery of the third transmission component, and an external thread structure is formed on the at least one first arc part of the sliding component.

Besides, the claimed auto-injector includes a reservoir, a plunger and any one of the aforementioned driving mechanisms.

In summary, in the present invention, the driving mechanism not only has compact structure and high power and high efficiency transmission but also achieves a long sliding distance and a slow sliding speed of the sliding component and prevents any rotation of the sliding component during a sliding movement of the sliding component. Therefore, the present invention can meet requirements of small volume, high driving power, long driving distance, long injecting period and accurate drug dose delivery rate.

These and other objectives of the present invention will no doubt become obvious to those of ordinary skill in the art after reading the following detailed description of the preferred embodiment that is illustrated in the various figures and drawings.

### Brief Description of the Drawings

In the following, the invention is further illustrated by way of example, taking reference to the accompanying drawings. Thereof
FIG. 1 and FIG. 2 are partial diagrams of an auto-injector at different views according to a first embodiment of the present invention,
FIG. 3 and FIG. 4 are diagrams of a driving mechanism at different views according to the first embodiment of the present invention,
FIG. 5 and FIG. 6 are partial diagrams of the driving mechanism at different views according to the first embodiment of the present invention,
FIG. 7 is another partial diagram of the driving mechanism according to the first embodiment of the present invention,
FIG. 8 and FIG. 9 are exploded diagrams of the driving mechanism at different views according to the first embodiment of the present invention,
FIG. 10 is a partial enlarged diagram of the driving mechanism according to the first embodiment of the present invention,
FIG. 11 is a functional block diagram of the driving mechanism according to the first embodiment of the present invention,
FIG. 12 and FIG. 13 are diagrams of the driving mechanism in different states according to the first embodiment of the present invention,
FIG. 14 is a diagram of a first transmission component according to a second embodiment of the present invention, and
FIG. 15 is a diagram of a first transmission component according to a third embodiment of the present invention.

### Detailed Description

In the following detailed description of the preferred embodiments, reference is made to the accompanying drawings which form a part hereof, and in which is shown by way of illustration specific embodiments in which the invention may be practiced. In this regard, directional terminology, such as "top", "bottom", "left", "right", "front", "back", etc., is used with reference to the orientation of the Figure(s) being described. The components of the present invention can be positioned in a number of different orientations. As such, the directional terminology is used for purposes of illustration and is in no way limiting. Accordingly, the drawings and descriptions will be regarded as illustrative in nature and not as restrictive. Also, if not specified, the term "connect" or "couple" is intended to mean either an indirect or direct electrical/mechanical connection. Thus, if a first device is connected or coupled to a second device, that connection may be through a direct electrical/mechanical connection, or through an indirect electrical/mechanical connection via other devices and connections.

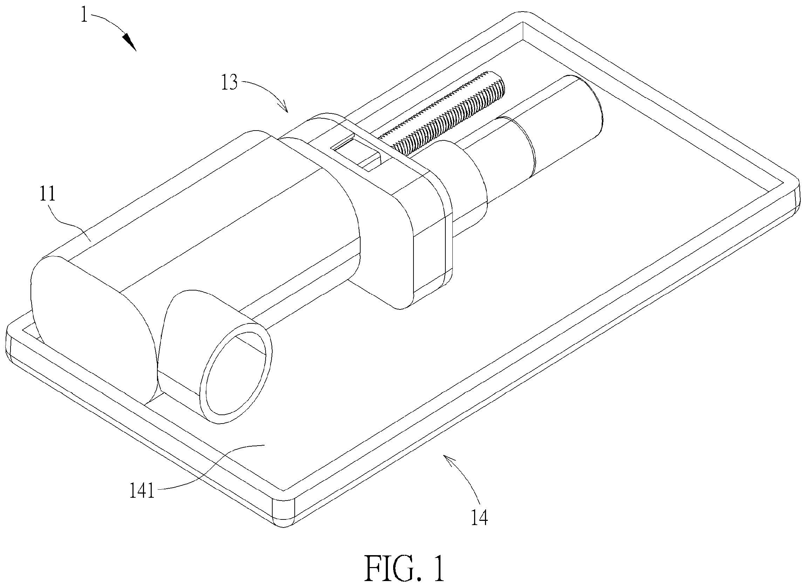

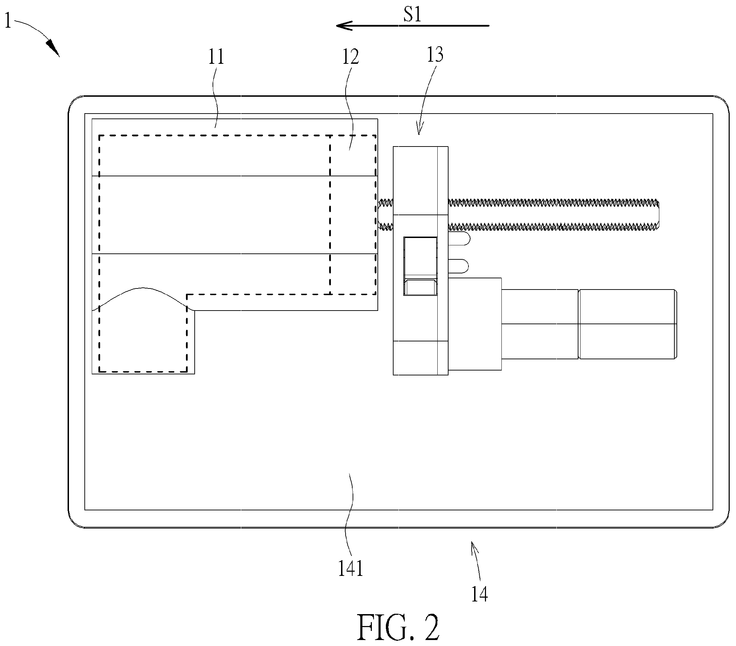

Please refer to FIG. 1 and FIG. 2. FIG. 1 and FIG. 2 are partial diagrams of an auto-injector 1 at different views according to a first embodiment of the present invention. As shown in FIG. 1 and FIG. 2, the auto-injector 1 includes a reservoir 11, a plunger 12 and a driving mechanism 13. The reservoir 11 is for drug storage. The plunger 12 is slidably disposed inside the reservoir 11. The driving mechanism 13 is for driving the plunger 12 to slide relative to the reservoir 11, so as to pump drug out of the reservoir 11 to complete a drug injection.

In this embodiment, the auto-injector 1 further includes a case 14. The case 14 includes a first mounting part 141, e.g., a lower shell, and a second mounting part, e.g., an upper shell, which is not shown in the figures, detachably installed on the first mounting part 141. The reservoir 11 and the driving mechanism 13 are mounted on the first mounting part 141. The case 14 is configured to conceal the reservoir 11 and the driving mechanism 13 for preventing damage of the reservoir 11 and the driving mechanism 13.

However, the present invention is not limited to this embodiment. For example, in another embodiment, the case can be a one-piece structure with an opening to at least partially reveal the reservoir and the driving mechanism.

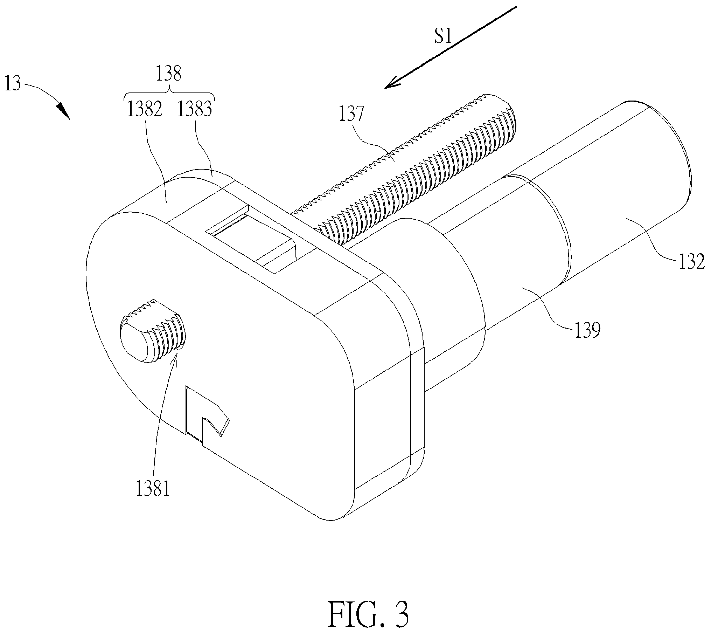

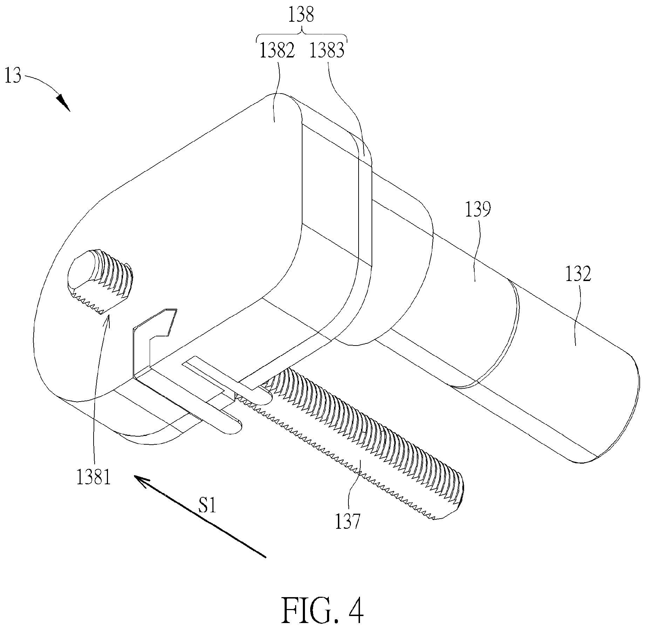

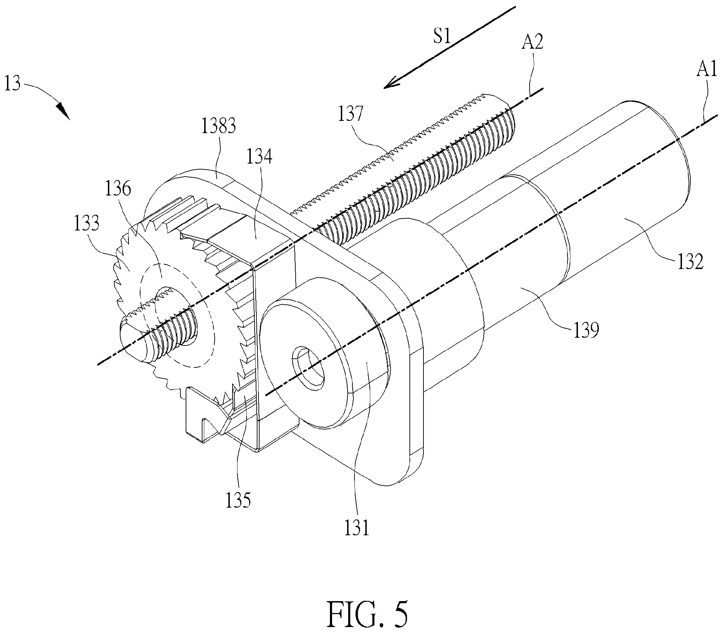

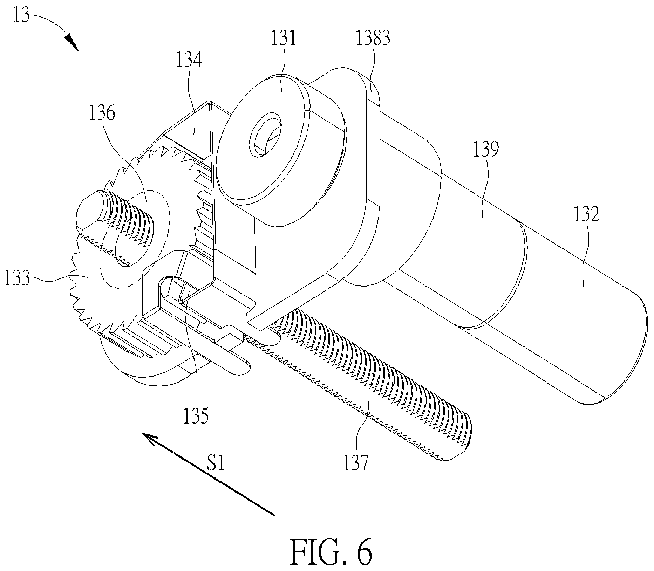

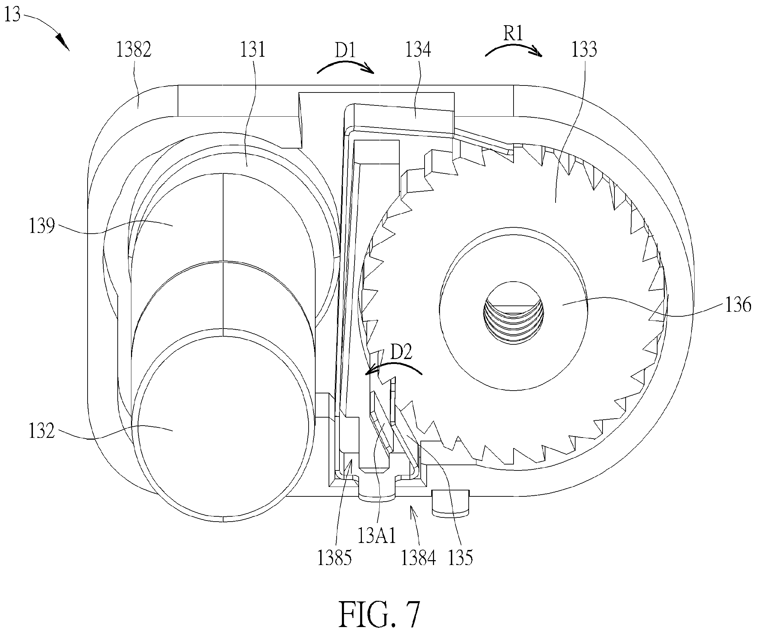

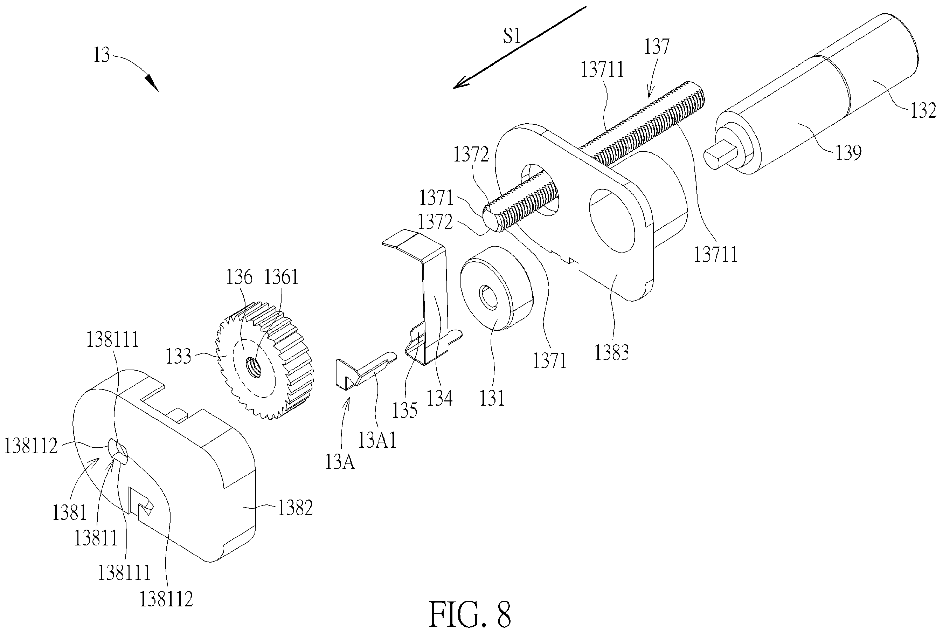

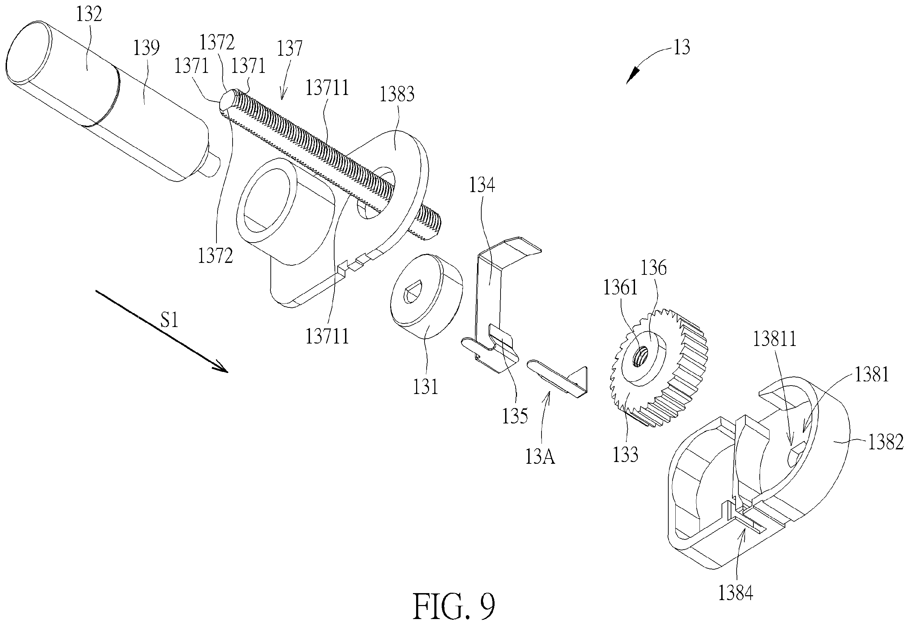

Please refer to FIG. 3 to FIG. 9. FIG. 3 and FIG. 4 are diagrams of the driving mechanism 13 at different views according to the first embodiment of the present invention. FIG. 5 and FIG. 6 are partial diagrams of the driving mechanism 13 at different views according to the first embodiment of the present invention. FIG. 7 is another partial diagram of the driving mechanism 13 according to the first embodiment of the present invention. FIG. 8 and FIG. 9 are exploded diagrams of the driving mechanism 13 at different views according to the first embodiment of the present invention. As shown in FIG. 3 to FIG. 9, the driving mechanism 13 includes a first transmission component 131, a driving component 132, a second transmission component 133, a driving resilient component 134, a stopping resilient component 135, a third transmission component 136, a sliding component 137 and a supporting component 138. The driving component 132 is coupled to the first transmission component 131 and for driving the first transmission component 131 to rotate. The second transmission component 133 is rotatably disposed apart from the first transmission component 131. The driving resilient component 134 is arranged between the first transmission component 131 and the second transmission component 133. The driving resilient component 134 is configured to be forced by the first transmission component 131 to resiliently deform to push the second transmission component 133 to rotate along a first rotating direction R1, or the driving resilient component 134 is configured to be released to resiliently recover. The stopping resilient component 135 is disposed adjacent to an outer periphery of the second transmission component 133. The stopping resilient component 135 is configured to be forced by the second transmission component 133 to resiliently deform when the driving resilient component 134 is forced by the first transmission component 131 to resiliently deform to push the second transmission component 133 to rotate along the first rotating direction R1. The stopping resilient component 135 is further configured to engage with the second transmission component 133 for stopping the second transmission component 133 from rotating along a second rotating direction R2 opposite to the first rotating direction R1 when the driving resilient component 134 is released to resiliently recover.

The third transmission component 136 is fixedly connected to the second transmission component 133, e.g., by a tightly fitting manner or an integrally forming manner. The third transmission component 136 is configured to be driven by the second transmission component 133 to rotate together with the second transmission component 133 when the second transmission component 133 rotates. The sliding component 137 is at least partially slidably disposed inside the third transmission component 136 and movably engaged with the third transmission component 136. The sliding component 137 is configured to be driven by the third transmission component 136 to slide relative to the second transmission component 133 along a first sliding direction S1 when the third transmission component 136 is driven by the second transmission component 133 to rotate along the first sliding direction S1 together with the second transmission component 133. The supporting component 138 includes a guiding portion 1381. The sliding component 137 passes through the guiding portion 1381, and the guiding portion 1381 is configured to guide the sliding component 137 to slide along the first sliding direction S1 without any rotation.

As shown in FIG. 2, the sliding component 137 is connected to the plunger 12. When the sliding component 137 is driven by the third transmission component 136 to slide along the first sliding direction S1, the plunger 12 is driven to slide relative to the reservoir 11 along the first sliding direction S1, so as to pump the drug out of the reservoir 11 to complete the drug injection.

Specifically, the first transmission component 131 can be an eccentric cam component. The second transmission component 133 can be a ratchet component. The third transmission component 136 can be a screw sleeve. The sliding component 137 can be a screw rod, and the driving component 132 can be an electric motor. However, the present invention is not limited to this embodiment. It depends on practical demands. For example, in another embodiment, the driving component can be a pneumatic motor.

Besides, in this embodiment, as shown in FIG. 3 to FIG. 9, the driving mechanism 13 further includes a reducer 139 coupled between the driving component 132 and the first transmission component 131. An input shaft and an output shaft of the reducer 139 can be respectively connected to the driving component 132 and the first transmission component 131. The reducer 139 can have various reduction ratios to control a rotating speed of the first transmission component 131, so as to control a sliding speed of the sliding component 137.

Specifically, the reducer 139 can be a gearbox. However, the present invention is not limited to this embodiment. It depends on practical demands. For example, in another embodiment, the reducer 139 can be a pulley and belt system. Alternatively, in another embodiment, the reducer can be omitted.

It should be noted that the aforementioned configuration can not only have small occupied space and achieve high power and high efficiency transmission but also achieve adjustment of a rotating speed of the second transmission component 133 by adjusting a reduction ratio between the driving component 132 and the first transmission component 131, e.g., a gear ratio of the reducer 139.

In order to make structure of the driving mechanism reasonably compact, as shown in FIG. 3 to FIG. 9, a first rotating axis A1 of the first transmission component 131 is parallel to and offset from a second rotating axis A2 of the second transmission component 133, and the first sliding direction S1 is parallel to an extending direction of the second rotating axis A2 of the second transmission component 133. The supporting component 138 includes a first supporting part 1382 and a second supporting part 1383 detachably assembled with the first supporting part 1382. The first transmission component 131, the second transmission component 133 and the third transmission component 136 are accommodated inside the supporting component 138 and located between the first supporting part 1382 and the second supporting part 1383. The driving resilient component 134 and the stopping resilient component 135 are integrally connected to each other to form an integral resilient structure. As shown in FIG. 7 and FIG. 9, the supporting component 138 further includes a mounting portion 1384 formed on the first supporting part 1382 and for mounting the integral resilient structure. In this embodiment, a channel 1385 is formed on the mounting portion 1384. The integral resilient structure passes through the channel 1385, and the driving resilient component 134 and the stopping resilient component 135 are partially exposed out of the mounting portion 1384. As shown in FIG. 7, the driving resilient component 134 is configured to be resiliently deformed by the first transmission component 131 along a first deforming direction D1 identical to the first rotating direction R1, and the stopping resilient component 135 is configured to be resiliently deformed by the second transmission component 133 along a second deforming direction D2 opposite to the first deforming direction D1. However, the present invention is not limited to this embodiment. For example, in another embodiment, the driving resilient component and the stopping resilient component can be separated from each other and located opposite to each other relative to the second transmission component.

Furthermore, in this embodiment, a rotating direction of the first transmission component 131 is identical to a rotating direction of the second transmission component 133. However, the present invention is not limited to this embodiment. For example, in another embodiment, the rotating direction of the first transmission component can be opposite to the rotating direction of the second transmission component, i.e., the first transmission component still can resiliently deform the driving resilient component along the first deforming direction for pushing the second transmission component to rotate along the first rotating direction even when the first transmission component rotates along the second rotating direction. In other words, the present invention can always ensure the drug injection no matter whether the rotating direction of the first transmission component is identical to the rotating direction of the second transmission component or not.

Besides, in order to achieve configuration of the guiding portion 1381 to guide the sliding component 137 to slide without any rotation when the third transmission component 136 rotatably drives the sliding component 137 to slide, as shown in FIG. 8 to FIG. 9, the guiding portion 1381 is formed on the first supporting part 1382 and includes a sliding through hole structure 13811. The sliding component 137 slidably passes through the sliding through hole structure 13811. A cross section of the sliding component 137 matches with a cross section of the sliding through hole structure 13811. The sliding component 137 includes two first arc parts 1371 opposite to each other, and two first flat parts 1372 opposite to each other and connected to the two first arc parts 1371. The sliding through hole structure 13811 includes two second arc parts 138111 opposite to each other, and two second flat parts 138112 opposite to each other and connected to the two second arc parts 138111. The two second arc parts 138111 and the two second flat parts 138112 are respectively corresponding to the two first arc parts 1371 and the two first flat parts 1372. An internal thread structure 1361 is formed on an inner periphery of the third transmission component 136, e.g., by plastic injection molding or insert molding, and an outer thread structure 13711 is formed on each of the first arc parts 1371 of the sliding component 137. The aforementioned configuration can ensure no rotation of the sliding component 137 during a sliding movement of the sliding component 137 by a cooperation of the sliding component 137 and the sliding through hole structure 13811, so as to meet a requirement of accurate drug dose delivery rate.

However, the structures of the sliding component and the guiding portion are not limited to this embodiment. For example, in another embodiment, the sliding component can include only one first arc part and one first flat part connected to the first arc part, and the sliding through hole structure can include only one second arc parts and one second flat part connected to the second arc part.

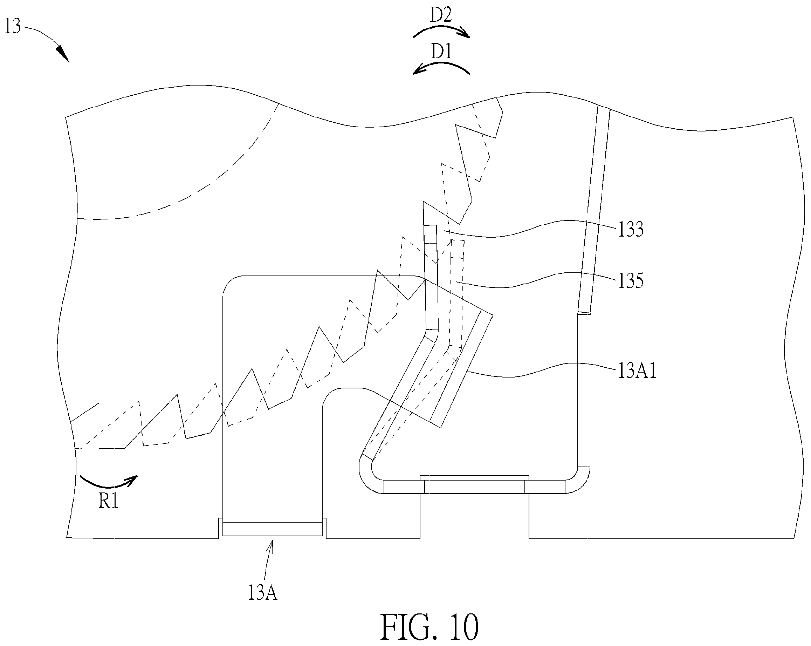

Please refer to FIG. 7 to FIG. 10. FIG. 10 is a partial enlarged diagram of the driving mechanism 13 according to the first embodiment of the present invention. As shown in FIG. 7 to FIG. 10, the driving mechanism 13 further includes a sensor 13A configured to sense a rotating movement of the second transmission component 133. In this embodiment, the sensor 13A includes an abutting component 13A1 disposed on the first supporting part 1382. The abutting component 13A1 is abutted by the stopping resilient component 135 when the stopping resilient component 135 is resiliently deformed by the second transmission component 133. Specifically, the abutting component 13A1 and the stopping resilient component 135 can respectively be two electrically conductive components electrically connected to a positive terminal and a negative terminal of an electric circuit. The electrical circuit can be closed for generating a first signal when the stopping resilient component 135 is resiliently deformed by the second transmission component 133 and abuts against the abutting component. The electrical circuit can be open for generating a second signal when the stopping resilient component 135 is released, e.g., separated away from the second transmission component 133, to resiliently recover and does not abut against the abutting component 13A1. However, the present invention is not limited to this embodiment. For example, in another embodiment, the sensor can be a non-contact sensor, e.g., a light sensor having a light receiver and a light emitter, and can be configured to generate the first signal when the stopping resilient component is resiliently deformed by the second transmission component to block or reflect light emitted from the light emitter.

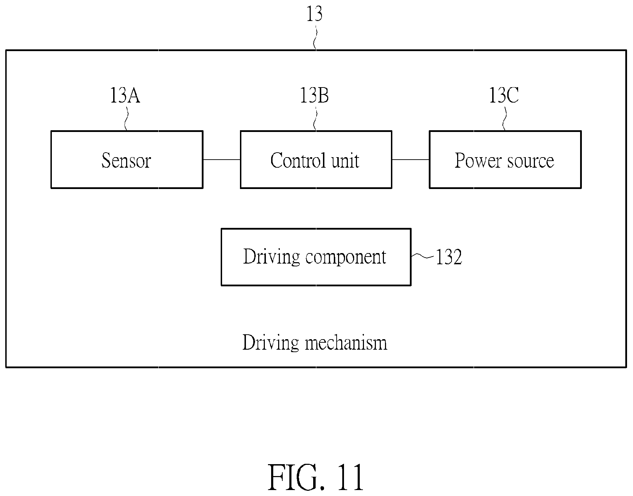

Please refer to FIG. 11. FIG. 11 is a functional block diagram of the driving mechanism 13 according to the first embodiment of the present invention. As shown in FIG. 11, the driving mechanism 13 further includes a control unit 13B and a power source 13C. The control unit 13B can be a circuit board electrically connected to the sensor 13A and the driving component 132 and configured to not only calculate a rotating speed, a number of rotation of the second transmission component 133 and/or a travel distance of the sliding component 137 according to the signals generated by the sensor 13A but also control the driving component 132 to actuate or stop a rotating movement of the first transmission component 131 and/or to control the rotating speed or a rotating direction of the first transmission component 131. The power source 13C can be a battery electrically connected to the control unit 13B and configured to provide electricity to the control unit 13B.

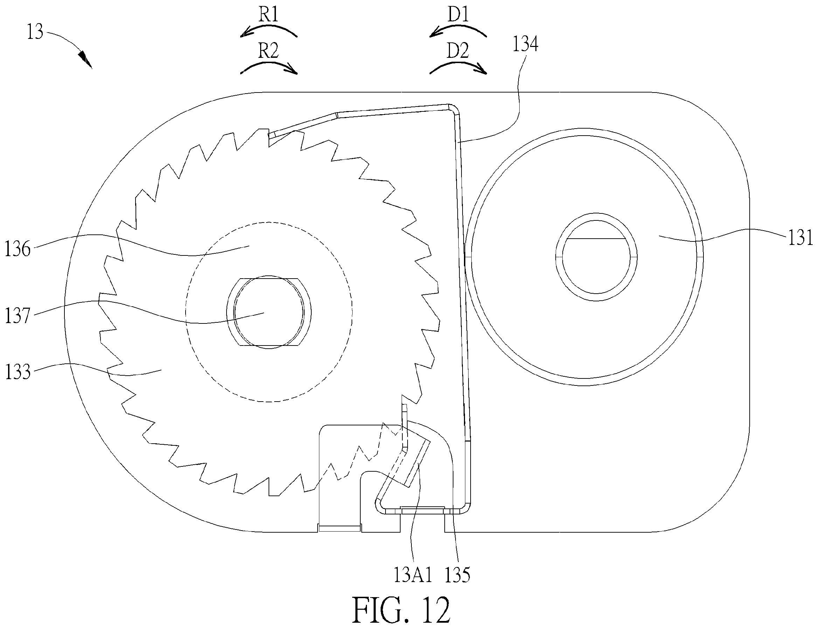

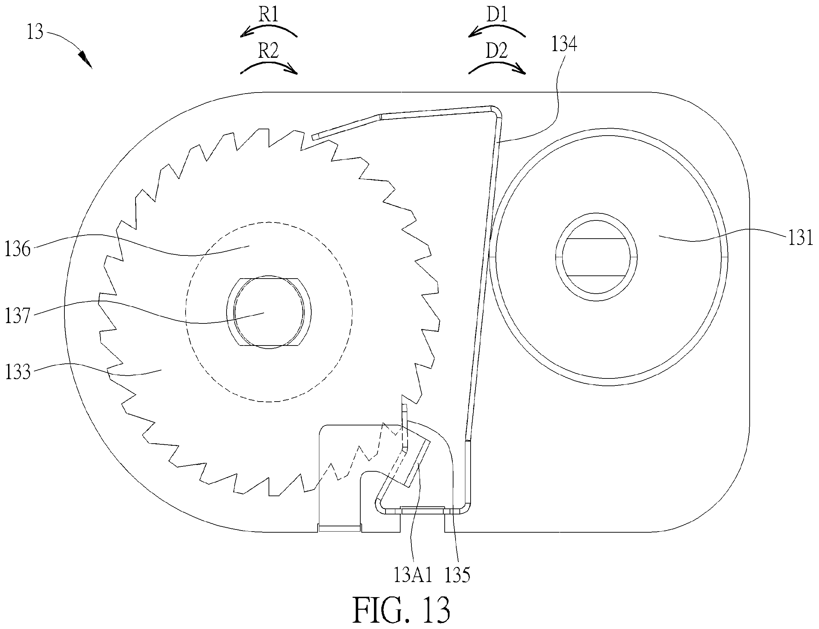

Detailed description for operational principle of the auto-injector 1 is provided as follows. Please further refer to FIG. 2, FIG. 5, FIG. 7, FIG. 10, FIG. 12 and FIG. 13. FIG. 12 and FIG. 13 are diagrams of the driving mechanism 13 in different states according to the first embodiment of the present invention. As shown in FIG. 2, FIG. 5, FIG. 7, FIG. 10, FIG. 12 and FIG. 13, after the auto-injector 1 is attached on a patient's body, the driving component 132 can be controlled by the control unit 13B to drive the first transmission component 131 to rotate around the first rotating axis A1 along the first rotating direction R1 with the reducer 139. When the first transmission component 131 is driven to rotate around the first rotating axis A1 along the first rotating direction R1, the driving resilient component 134 is forced by the first transmission component 131 to resiliently deform along the first deforming direction D1 to push the second transmission component 133 to rotate along the first rotating direction R1 and then released to resiliently recover along the second deforming direction D2 repeatedly. When the second transmission component 133 rotates around the second rotating axis A2 along the first rotating direction R1, the third transmission component 136 rotates around the second rotating axis A2 along the first rotating direction R1 together with the second transmission component 133, so that the sliding component 137 is driven to slide relative to the third transmission component 136 along the first sliding direction S1 without any rotation, so as to drive the plunger 12 to slide relative to the reservoir 11 along the first sliding direction S1 for pumping drug out of the reservoir 11.

Specifically, in each rotation cycle of the first transmission component 131, the driving resilient component 134 is moved from a position as shown in FIG. 13 to a position as shown in FIG. 12 and then back to the position as shown in FIG. 13. When the driving resilient component 134 is moved from the position as shown in FIG. 13 to the position as shown in FIG. 12, the second transmission component 133 is driven by the driving resilient component 134 to rotate along the first rotating direction R1 at a predetermined rotation angle. During the aforementioned process, as shown in FIG. 10, the stopping resilient component 135 is resiliently deformed along the second deforming direction D2 by the second transmission component 133 and then released to recover along first deforming direction D1. Afterwards, when the driving resilient component 134 is recovered from the position as shown in FIG. 12 back to the position as shown in FIG. 13, the recovered stopping resilient component 135 engages with the second transmission component 133 for preventing the recovered driving resilient component 134 from driving the second transmission component 133 to rotate along the second rotating direction R2, so that the second transmission component 133 is stopped.

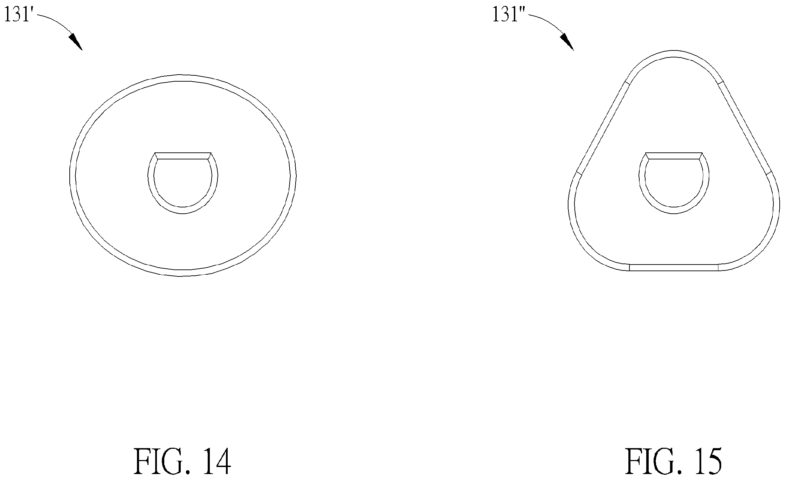

Understandably, a shape of the first transmission component 131 can be determined according to practical demands, and it decides a driving frequency of the driving resilient component 134 for driving the second transmission component 133 to rotate along the first rotating direction R1 in each rotation cycle of the first transmission component 131. For example, please refer to FIG. 14 and FIG. 15. FIG. 14 is a diagram of a first transmission component 131' according to a second embodiment of the present invention. FIG. 15 is a diagram of a first transmission component 131" according to a third embodiment of the present invention. As shown in FIG. 14, in the second embodiment, the first transmission component 131' can be an ellipse cam component, which can push the driving resilient component twice in each rotation cycle of the first transmission component 131'. As shown in FIG. 15, in the third embodiment, the first transmission component 131" can be a triangular cam component, which can push the driving resilient component three times in each rotation cycle of the first transmission component 131".

Besides, understandably, a circular pitch of the second transmission component 133 also can be determined according to practical demands, which decides the predetermined rotation angle of the second transmission component 133 in each rotation cycle of the first transmission component 131.

In contrast to the prior art, in the present invention, the driving mechanism not only has compact structure and high power and high efficiency transmission but also achieves a long sliding distance and a slow sliding speed of the sliding component and prevents any rotation of the sliding component during a sliding movement of the sliding component. Therefore, the present invention can meet requirements of small volume, high driving power, long driving distance, long injecting period and accurate drug dose delivery rate.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. A driving mechanism (13) for driving a plunger (12) of an auto-injector (1) to slide relative to a reservoir (11) of the auto-injector (1), the driving mechanism (13) comprising:
a first transmission component (131, 131', 131");
a driving component (132) coupled to the first transmission component (131, 131', 131") and for driving the first transmission component (131, 131', 131") to rotate;
a second transmission component (133) rotatably disposed apart from the first transmission component (131, 131', 131");
a driving resilient component (134) arranged between the first transmission component (131, 131', 131") and the second transmission component (133), the driving resilient component (134) being configured to be forced by the first transmission component (131, 131', 131") to resiliently deform to push the second transmission component (133) to rotate along a first rotating direction (R1), or the driving resilient component being configured to be released to resiliently recover;
a stopping resilient component (135) disposed adjacent to an outer periphery of the second transmission component (133), the stopping resilient component (135) being forced by the second transmission component (133) to resiliently deform when the driving resilient component (134) is forced by the first transmission component (131, 131', 131") to resiliently deform to push the second transmission component (133) to rotate along the first rotating direction (R1), the stopping resilient component (135) engaging with the second transmission component (133) for stopping the second transmission component (133) from rotating along a second rotating direction (R2) opposite to the first rotating direction (R1) when the driving resilient component (134) is released to resiliently recover;
a third transmission component (136) fixedly connected to the second transmission component (133), the third transmission component (136) being driven by the second transmission component (133) to rotate together with the second transmission component (133) when the second transmission component (133) rotates;
a sliding component (137) at least partially slidably disposed inside the third transmission component (136) and movably engaged with the third transmission component (136), the sliding component (137) being connected to the plunger (12), the sliding component (137) being driven by the third transmission component (136) to slide relative to the second transmission component (133) along a first sliding direction (S1) when the third transmission component (136) is driven by the second transmission component (133) to rotate along the first sliding direction (S1) together with the second transmission component (133); and
a supporting component (138) comprising a guiding portion (1381), the sliding component (137) passing through the guiding portion (1381), and the guiding portion (1381) being configured to guide the sliding component (137) to slide along the first sliding direction (S1) without rotation.

2. The driving mechanism (13) of claim 1, further **characterized by** a sensor (13A) configured to sense a rotating movement of the second transmission component (133).

3. The driving mechanism (13) of claim 2, **characterized in that** the sensor (13A) comprises an abutting component (13A1), the abutting component (13A1) is abutted by the stopping resilient component (135) when the stopping resilient component (135) is resiliently deformed by the second transmission component (133).

4. The driving mechanism (13) of any one of claims 1 to 3, **characterized in that** the driving resilient component (134) and the stopping resilient component (135) are integrally connected to each other to form an integral resilient structure.

5. The driving mechanism (13) of claim 4, **characterized in that** the supporting component (138) further comprises a mounting portion (1384) for mounting the integral resilient structure.

6. The driving mechanism (13) of claim 5, **characterized in that** a channel (1385) is formed on the mounting portion (1384), the integral resilient structure passes through the channel (1385), and the driving resilient component (134) and the stopping resilient component (135) are partially exposed out of the mounting portion (1384).

7. The driving mechanism (13) of any one of claims 1 to 6, **characterized in that** the driving resilient component (134) is resiliently deformed by the first transmission component (131, 131', 131") along a first deforming direction (D1) identical to the first rotating direction (R1), and the stopping resilient component (135) is resiliently deformed by the second transmission component (133) along a second deforming direction (D2) opposite to the first deforming direction (D1).

8. The driving mechanism (13) of any one of claims 1 to 7, **characterized in that** the first transmission component (131, 131', 131"), the second transmission component (133) and the third transmission component (136) are accommodated inside the supporting component (138).

9. The driving mechanism (13) of any one of claims 1 to 8, **characterized in that** a first rotating axis (A1) of the first transmission component (131, 131', 131") is parallel to a second rotating axis (A2) of the second transmission component (133), and the first sliding direction (S1) is parallel to an extending direction of the second rotating axis (A2) of the second transmission component (133).

10. The driving mechanism (13) of any one of claims 1 to 9, **characterized in that** the first transmission component (131, 131', 131") is a cam component, the second transmission component (133) is a ratchet component, the third transmission component (136) is a screw sleeve, the sliding component (137) is a screw rod, and the driving component (132) is an electric motor.

11. The driving mechanism (13) of any one of claims 1 to 10, further **characterized by** a reducer (139) coupled between the driving component (132) and the first transmission component (131, 131', 131").

12. The driving mechanism (13) of claim 11, **characterized in that** the reducer (139) is a gearbox.

13. The driving mechanism (13) of any one of claims 1 to 12, **characterized in that** the guiding portion (1381) comprises a sliding through hole structure (13811), the sliding component (137) slidably passes through the sliding through hole structure (13811), a cross section of the sliding component (137) matches with a cross section of the sliding through hole structure (13811), the sliding component (137) comprises at least one first arc part (1371) and at least one first flat part (1372) connected to the at least one first arc part (1371), the sliding through hole structure (13811) comprises at least one second arc part (138111) and at least one second flat part (138112) connected to the at least one second arc part (138111), and the at least one second arc part (138111) and the at least one second flat part (138112) are arranged respectively corresponding to the at least one first arc part (1371) and the at least one first flat part (1372).

14. The driving mechanism (13) of claim 13, **characterized in that** an internal thread structure (1361) is formed on an inner periphery of the third transmission component (136), and an external thread structure (13711) is formed on the at least one first arc part (1371) of the sliding component (137).

15. An auto-injector (1) comprising:
a reservoir (11); and
a plunger (12) slidably disposed inside the reservoir (11); and
**characterized in that** the auto-injector (1) further comprises:
the driving mechanism (13) of any one of claims 1 to 14.

## Patentansprüche

1. Antriebsmechanismus (13) zum Antreiben eines Kolbens (12) eines Autoinjektors (1), damit dieser relativ zu einem Reservoir (11) des Autoinjektors (1) gleitet, wobei der Antriebsmechanismus (13) umfasst:
eine erste Übertragungskomponente (131, 131', 131");
eine Antriebskomponente (132), die mit der ersten Übertragungskomponente (131, 131', 131") gekoppelt ist und zum Antreiben der ersten Übertragungskomponente (131, 131', 131") zur Drehung dient;
eine zweite Übertragungskomponente (133), die drehbar von der ersten Übertragungskomponente (131, 131', 131") beabstandet angeordnet ist;
eine elastische Antriebskomponente (134), die zwischen der ersten Übertragungskomponente (131, 131', 131") und der zweiten Übertragungskomponente (133) angeordnet ist, wobei die elastische Antriebskomponente (134) ausgestaltet ist, von der ersten Übertragungskomponente (131, 131', 131") elastisch verformt zu werden, um die zweite Übertragungskomponente (133) zu einer Drehung entlang einer ersten Drehrichtung (R1) zu zwingen, oder wobei die elastische Antriebskomponente ausgestaltet ist, freigegeben zu werden, um sich elastisch zurückzubilden;
eine an einem Außenumfang der zweiten Übertragungskomponente (133) angeordnete elastische Anschlagkomponente (135), wobei die elastische Anschlagkomponente (135) durch die zweite Übertragungskomponente (133) zu einer elastischen Verformung gezwungen wird, wenn die elastische Antriebskomponente (134) durch die erste Übertragungskomponente (131, 131', 131") elastisch verformt wird, um die zweite Übertragungskomponente (133) zu einer Drehung entlang der ersten Drehrichtung (R1) zu zwingen, wobei die elastische Anschlagkomponente (135) mit der zweiten Übertragungskomponente (133) in Eingriff steht, um die zweite Übertragungskomponente (133) daran zu hindern, sich entlang einer zweiten Drehrichtung (R2) entgegengesetzt zur ersten Drehrichtung (R1) zu drehen, wenn die elastische Antriebskomponente (134) freigegeben wird, um sich elastisch zurückzubilden;
eine dritte Übertragungskomponente (136), die fest mit der zweiten Übertragungskomponente (133) verbunden ist, wobei die dritte Übertragungskomponente (136) von der zweiten Übertragungskomponente (133) angetrieben wird, um sich zusammen mit der zweiten Übertragungskomponente (133) zu drehen, wenn sich die zweite Übertragungskomponente (133) dreht;
eine Gleitkomponente (137), die zumindest teilweise gleitend innerhalb der dritten Übertragungskomponente (136) angeordnet ist und beweglich mit der dritten Übertragungskomponente (136) in Eingriff steht, worin die Gleitkomponente (137) mit dem Kolben (12) verbunden ist, wobei die Gleitkomponente (137) von der dritten Übertragungskomponente (136) angetrieben wird, um relativ zur zweiten Übertragungskomponente (133) entlang einer ersten Gleitrichtung (S1) zu gleiten, wenn die dritte Übertragungskomponente (136) von der zweiten Übertragungskomponente (133) angetrieben wird, um sich zusammen mit der zweiten Übertragungskomponente (133) entlang der ersten Gleitrichtung (S1) zu drehen; und
eine Stützkomponente (138) mit einem Führungsabschnitt (1381), worin die Gleitkomponente (137) durch den Führungsabschnitt (1381) hindurchgeht und der Führungsabschnitt (1381) ausgestaltet ist, die Gleitkomponente (137) so zu führen, dass diese ohne Drehung entlang der ersten Gleitrichtung (S1) gleitet.

2. Antriebsmechanismus (13) nach Anspruch 1, ferner **gekennzeichnet durch** einen Sensor (13A), der ausgestaltet ist, eine Drehbewegung der zweiten Übertragungskomponente (133) zu erfassen.

3. Antriebsmechanismus (13) nach Anspruch 2, **dadurch gekennzeichnet, dass** der Sensor (13A) eine Anschlagkomponente (13A1) umfasst, wobei die Anschlagkomponente (13A1) von der elastische Anschlagkomponente (135) zum Anliegen gebracht wird, wenn die elastische Anschlagkomponente (135) durch die zweite Übertragungskomponente (133) elastisch verformt wird.

4. Antriebsmechanismus (13) nach einem der Ansprüche 1 bis 3, **dadurch gekennzeichnet, dass** die elastische Antriebskomponente (134) und die elastische Anschlagkomponente (135) einstückig miteinander verbunden sind, um eine einstückig Federstruktur auszubilden.

5. Antriebsmechanismus (13) nach Anspruch 4, **dadurch gekennzeichnet, dass** die Stützkomponente (138) ferner einen Befestigungsabschnitt (1384) zum Befestigen der einstückigen elastischen Struktur umfasst.

6. Antriebsmechanismus (13) nach Anspruch 5, **dadurch gekennzeichnet, dass** an dem Befestigungsabschnitt (1384) ein Kanal (1385) ausgebildet ist, die einstückige elastische Struktur durch den Kanal (1385) verläuft und die elastische Antriebskomponente (134) und die elastische Anschlagkomponente (135) teilweise aus dem Befestigungsabschnitt (1384) herausragen.

7. Antriebsmechanismus (13) nach einem der Ansprüche 1 bis 6, **dadurch gekennzeichnet, dass** die elastische Antriebskomponente (134) durch die erste Übertragungskomponente (131, 131', 131") entlang einer ersten Verformungsrichtung (D1) elastisch verformt wird, die mit der ersten Drehrichtung (R1) identisch ist, und die elastische Anschlagkomponente (135) durch die zweite Übertragungskomponente (133) entlang einer zweiten Verformungsrichtung (D2) elastisch verformt wird, die der ersten Verformungsrichtung (D1) entgegengesetzt ist.

8. Antriebsmechanismus (13) nach einem der Ansprüche 1 bis 7, **dadurch gekennzeichnet, dass** die erste Übertragungskomponente (131, 131', 131"), die zweite Übertragungskomponente (133) und die dritte Übertragungskomponente (136) innerhalb der Stützkomponente (138) aufgenommen sind.

9. Antriebsmechanismus (13) nach einem der Ansprüche 1 bis 8, **dadurch gekennzeichnet, dass** eine erste Drehachse (A1) der ersten Übertragungskomponente (131, 131', 131") parallel zu einer zweiten Drehachse (A2) der zweiten Übertragungskomponente (133) ist und die erste Gleitrichtung (S1) parallel zu einer Erstreckungsrichtung der zweiten Drehachse (A2) der zweiten Übertragungskomponente (133) ist.

10. Antriebsmechanismus (13) nach einem der Ansprüche 1 bis 9, **dadurch gekennzeichnet, dass** die erste Übertragungskomponente (131, 131', 131") eine Nockenkomponente ist, die zweite Übertragungskomponente (133) eine Ratschenkomponente ist, die dritte Übertragungskomponente (136) eine Schraubhülse ist, die Gleitkomponente (137) eine Schraubstange ist und die Antriebskomponente (132) ein Elektromotor ist.

11. Antriebsmechanismus (13) nach einem der Ansprüche 1 bis 10, ferner **gekennzeichnet durch** eine Untersetzung (139), die zwischen der Antriebskomponente (132) und der ersten Übertragungskomponente (131, 131', 131") gekoppelt ist.

12. Antriebsmechanismus (13) nach Anspruch 11, **dadurch gekennzeichnet, dass** die Untersetzung (139) ein Getriebe ist.

13. Antriebsmechanismus (13) nach einem der Ansprüche 1 bis 12, **dadurch gekennzeichnet, dass** der Führungsabschnitt (1381) eine Gleitdurchgangslochstruktur (13811) umfasst, die Gleitkomponente (137) gleitend durch die Gleitdurchgangslochstruktur (13811) hindurchgeht, ein Querschnitt der Gleitkomponente (137) mit einem Querschnitt der Gleitdurchgangslochstruktur (13811) übereinstimmt, die Gleitkomponente (137) mindestens einen ersten Bogenteil (1371) und mindestens einen ersten Flachteil (1372) umfasst, der mit dem mindestens einen ersten Bogenteil (1371) verbunden ist, die Gleitdurchgangslochstruktur (13811) mindestens einen zweiten Bogenteil (138111) und mindestens einen zweiten Flachteil (138112) umfasst, der mit dem mindestens einen zweiten Bogenteil (138111) verbunden ist, und der mindestens eine zweite Bogenteil (138111) und der mindestens eine zweite Flachteil (138112) entsprechend dem mindestens einen ersten Bogenteil (1371) und dem mindestens einen ersten Flachteil (1372) angeordnet sind.

14. Antriebsmechanismus (13) nach Anspruch 13, **dadurch gekennzeichnet, dass** eine Innengewindestruktur (1361) an einem Innenumfang der dritten Übertragungskomponente (136) ausgebildet ist und eine Außengewindestruktur (13711) an dem mindestens einen ersten Bogenteil (1371) der Gleitkomponente (137) ausgebildet ist.

15. Autoinjektor (1), welcher umfasst:
ein Reservoir (11); und
einen Kolben (12), der verschiebbar innerhalb des Reservoirs (11) angeordnet ist; und
**dadurch gekennzeichnet, dass** der Autoinjektor (1) ferner umfasst:
den Antriebsmechanismus (13) nach einem der Ansprüche 1 bis 14.

## Revendications

1. Mécanisme d'entraînement (13) destiné à entraîner un piston (12) d'un auto-injecteur (1) afin qu'il coulisse par rapport à un réservoir (11) de l'auto-injecteur (1), le mécanisme d'entraînement (13) comprenant:
un premier composant de transmission (131, 131', 131");
un composant d'entraînement (132) couplé au premier composant de transmission (131, 131', 131'") et destiné à entraîner le premier composant de transmission (131, 131", 131") en rotation;
un deuxième composant de transmission (133) disposé de manière rotative à distance du premier composant de transmission (131, 131', 131");
un composant élastique d'entraînement (134) disposé entre le premier composant de transmission (131, 131', 131''') et le deuxième composant de transmission (133), le composant élastique d'entraînement (134) étant configuré pour être forcé par le premier composant de transmission (131, 131", 131") à se déformer de manière élastique afin de pousser le deuxième composant de transmission (133) à tourner selon un premier sens de rotation (R1), ou le composant élastique d'entraînement étant configuré pour être relâché afin de se rétablir de manière élastique;
un composant élastique d'arrêt (135) disposé à proximité d'une périphérie extérieure du deuxième composant de transmission (133), le composant élastique d'arrêt (135) étant contraint par le deuxième composant de transmission (133) à se déformer de manière élastique lorsque le composant élastique d'entraînement (134) est contraint par le premier composant de transmission (131, 131', 131") à se déformer élastiquement pour pousser le deuxième composant de transmission (133) à tourner dans le premier sens de rotation (R1), le composant élastique d'arrêt (135) s'engageant avec le deuxième composant de transmission (133) pour empêcher le deuxième composant de transmission (133) de tourner dans un deuxième sens de rotation (R2) opposé au premier sens de rotation (R1) lorsque le composant élastique d'entraînement (134) est relâché pour se rétablir de manière élastique;
un troisième composant de transmission (136) relié de manière fixe au deuxième composant de transmission (133), le troisième composant de transmission (136) étant entraîné par le deuxième composant de transmission (133) pour tourner avec le deuxième composant de transmission (133) lorsque le deuxième composant de transmission (133) tourne;
un composant coulissant (137) disposé au moins partiellement de manière coulissante à l'intérieur du troisième composant de transmission (136) et engagé de manière mobile avec le troisième composant de transmission (136), le composant coulissant (137) étant relié au piston (12), le composant coulissant (137) étant entraîné par le troisième composant de transmission (136) pour coulisser par rapport au deuxième composant de transmission (133) le long d'une première direction de coulissement (S1) lorsque le troisième composant de transmission (136) est entraîné par le deuxième composant de transmission (133) pour tourner le long de la première direction de coulissement (S1) avec le deuxième composant de transmission (133); et
un composant de support (138) comprenant une partie de guidage (1381), le composant coulissant (137) passant à travers la partie de guidage (1381), et la partie de guidage (1381) étant configurée pour guider le composant coulissant (137) afin qu'il coulisse le long de la première direction de coulissement (S1) sans rotation.

2. Mécanisme d'entraînement (13) selon la revendication 1, **caractérisé en outre par** un capteur (13A) configuré pour détecter un mouvement de rotation du deuxième composant de transmission (133).

3. Mécanisme d'entraînement (13) selon la revendication 2, **caractérisé en ce que** le capteur (13A) comprend un composant de butée (13A1), le composant de butée (13A1) étant en butée contre le composant élastique d'arrêt (135) lorsque le composant élastique d'arrêt (135) est déformé de manière élastique par le deuxième composant de transmission (133).

4. Mécanisme d'entraînement (13) de l'une quelconque des revendications 1 à 3, **caractérisé en ce que** le composant élastique d'entraînement (134) et le composant élastique d'arrêt (135) sont reliés entre eux de manière intégrale pour former une structure élastique intégrale.

5. Mécanisme d'entraînement (13) selon la revendication 4, **caractérisé en ce que** le composant de support (138) comprend en outre une partie de montage (1384) pour monter la structure élastique intégrale.

6. Mécanisme d'entraînement (13) selon la revendication 5, **caractérisé en ce qu'**un canal (1385) est formé sur la partie de montage (1384), la structure élastique intégrale passe à travers le canal (1385), et le composant élastique d'entraînement (134) et le composant élastique d'arrêt (135) sont partiellement exposés à l'extérieur de la partie de montage (1384).

7. Mécanisme d'entraînement (13) selon l'une quelconque des revendications 1 à 6, **caractérisé en ce que** le composant élastique d'entraînement (134) est déformé de manière élastique par le premier composant de transmission (131, 131', 131") le long d'une première direction de déformation (D1) identique à la première direction de rotation (R1), et le composant élastique d'arrêt (135) est déformé élastiquement par le deuxième composant de transmission (133) le long d'une deuxième direction de déformation (D2) opposée à la première direction de déformation (D1).

8. Mécanisme d'entraînement (13) de l'une quelconque des revendications 1 à 7, **caractérisé en ce que** le premier composant de transmission (131, 131', 131"), le deuxième composant de transmission (133) et le troisième composant de transmission (136) sont logés à l'intérieur du composant de support (138).

9. Mécanisme d'entraînement (13) selon l'une quelconque des revendications 1 à 8, **caractérisé en ce qu'**un premier axe de rotation (A1) du premier composant de transmission (131, 131', 131") est parallèle à un deuxième axe de rotation (A2) du deuxième composant de transmission (133), et la première direction de glissement (S1) est parallèle à une direction d'extension du deuxième axe de rotation (A2) du deuxième composant de transmission (133).

10. Mécanisme d'entraînement (13) de l'une quelconque des revendications 1 à 9, **caractérisé en ce que** le premier composant de transmission (131, 131', 131") est un composant à came, le deuxième composant de transmission (133) est un composant à cliquet, le troisième composant de transmission (136) est un manchon à vis, le composant coulissant (137) est une tige à vis, et le composant d'entraînement (132) est un moteur électrique.

11. Mécanisme d'entraînement (13) selon l'une quelconque des revendications 1 à 10, **caractérisé en outre par** un réducteur (139) couplé entre le composant d'entraînement (132) et le premier composant de transmission (131, 131', 131").

12. Mécanisme d'entraînement (13) selon la revendication 11, **caractérisé en ce que** le réducteur (139) est une boîte de vitesses.

13. Mécanisme d'entraînement (13) de l'une quelconque des revendications 1 à 12, **caractérisé en ce que** la partie de guidage (1381) comprend une structure à trou traversant coulissant (13811), le composant coulissant (137) passe de manière coulissante à travers la structure à trou traversant coulissant (13811), une section transversale du composant coulissant (137) correspond à une section transversale de la structure à trou traversant coulissant (13811), le composant coulissant (137) comprend au moins une première partie arquée (1371) et au moins une première partie plate (1372) reliée à l'au moins une première partie arquée (1371), la structure à trou traversant coulissant (13811) comprend au moins une deuxième partie arquée (138111) et au moins une deuxième partie plate (138112) reliée à l'au moins une deuxième partie arquée (138111), et l'au moins une deuxième partie arquée (138111) et l'au moins une deuxième partie plate (138112) sont disposées respectivement en correspondance avec l'au moins une première partie arquée (1371) et l'au moins une première partie plate (1372).

14. Mécanisme d'entraînement (13) selon la revendication 13, **caractérisé en ce qu'**une structure filetée interne (1361) est formée sur une périphérie interne du troisième composant de transmission (136), et une structure filetée externe (13711) est formée sur au moins une première partie arquée (1371) du composant coulissant (137).

15. Auto-injecteur (1) comprenant:
un réservoir (11); et
un piston (12) disposé de manière coulissante à l'intérieur du réservoir (11); et
**caractérisé en ce que** l'auto-injecteur (1) comprend en outre:
le mécanisme d'entraînement (13) de l'une quelconque des revendications 1 à 14.
